# EUROPEAN PATENT APPLICATION

(11) **EP 3 991 735 A1**
(43) Date of publication of application: **04.05.2022**
(21) Application number: 21205318.5
(22) Date of filing: 28.10.2021
(51) Int. Cl.: A61K 31/7032, A61K 36/85, A61K 36/28, A61P 5/24, A61K 31/216, A61K 36/53

(54) **PREPARATION FOR THE TREATMENT OF MALE INFERTILITY**

(30) Priority: 02.11.2020 IT 202000026038
(71) Applicant: Abresearch S.r.l., 36040 Brendola (Vicenza) (IT)
(72) Inventor: DAL MONTE, Renzo, I-36040 BRENDOLA, VICENZA (IT); COSENTINO, Marco, I-36040 BRENDOLA, VICENZA (IT); BEDIN, Chiara, I-36040 BRENDOLA, VICENZA (IT); SACCANI, Andrea, I-36040 BRENDOLA, VICENZA (IT); CARPI, Andrea, I-36040 BRENDOLA, VICENZA (IT)
(74) Representative: Croce, Valeria

(57) **Abstract**

The present invention relates to a preparation based on compounds of vegetable origin for the treatment of male infertility.

## Description

### Technical field of the invention

The present invention applies to the pharmaceutical and medical fields, and in particular, relates to a preparation for the treatment of infertility.

### Background art

Over the past 30 years, a significant increase in infertility has been noted and, in about 50% of involuntarily infertile couples, male infertility is usually associated with abnormal sperm parameters.

About 15% of couples do not reach pregnancy within a year and seek medical treatment for infertility: for years, antioxidant therapies have been commonly prescribed or purchased directly by subjects without a doctor's prescription due to the well-known effect on sperm quality.

The scientific community agrees that oxidative stress plays a fundamental role in male infertility, with spermatozoa being the first cells to be described as susceptible to oxidative stress.

In the context of infertility, an obstructive cause can also be established (defined as crypto-/azoospermia), in some cases represented by a temporary obstruction caused by a reduction of the duct lumen following inflammation; in particular, the causes can be:
- obstructive (OA): when the production of spermatozoa is normal, but there is an obstruction (epididymal/deferential/distal) which makes the transit of gametes in the seminal fluid impossible; or
- secretory or non-obstructive (NOA): when there is a defect in spermatogenesis or spermiogenesis, on a genetic or acquired basis, such as to compromise the production of male gametes.

Therefore, the treatment of inflammation can help solve such a problem.

Various antioxidants have been suggested for the treatment of male infertility over the years, but none of these have shown particularly advantageous results.

The common therapy includes administering antioxidant supplements such as carnitine, coenzyme Q10, vitamin C, vitamin E, zinc, folic acid, N-acetylcysteine, selenium alone, or a multivitamin mixture.

Traditional Chinese medicine uses *Cistanche tubulosa* (CT) to increase male sexual function.

One of the main compounds thereof is represented by echinacoside which, being known to improve the quality of sperm, is a natural reproductive agent.

The market pushes to continuously search for the best options for patients.

### Summary of the invention

The authors of the present invention have surprisingly found that an appropriate combination of verbascoside, teupolioside and echinacoside is capable of surprisingly improving the properties of sperm, so as to increase the probability of spontaneous pregnancy.

### Brief description of the drawings

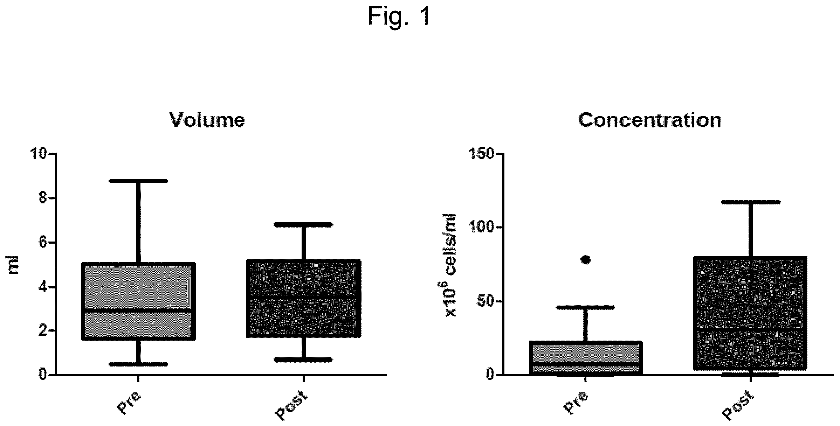

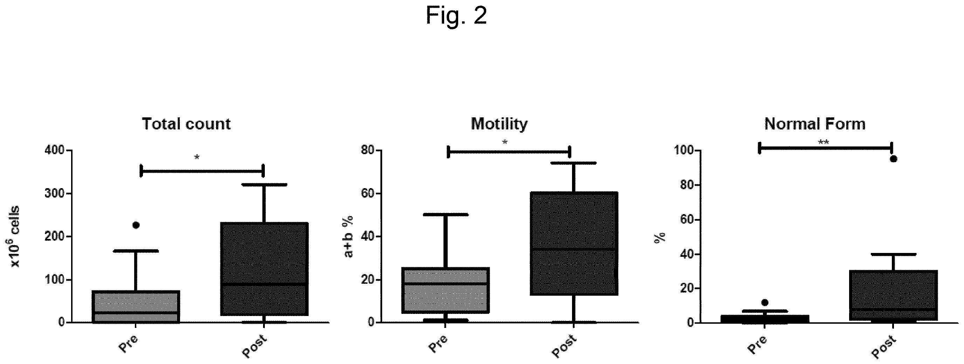

Figures 1 and 2 show the graphs of the distribution of the data reported in Table 1 between pre- and post-treatment of the single seminal parameters considered and levels of significance (p>0.05=*; p>0.001=**);

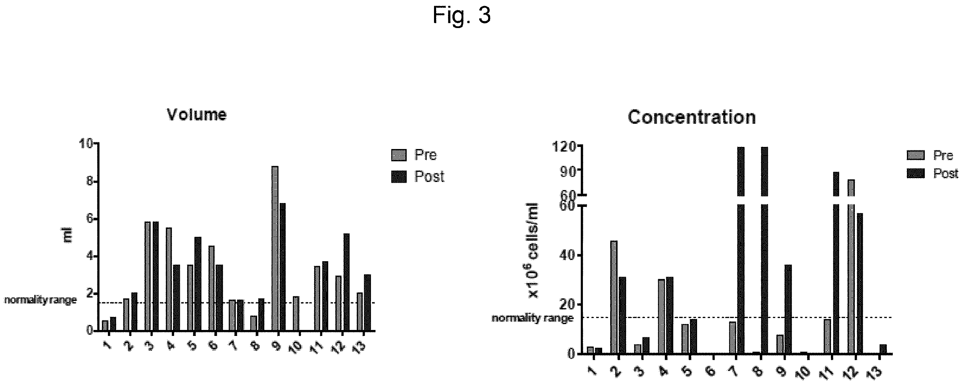

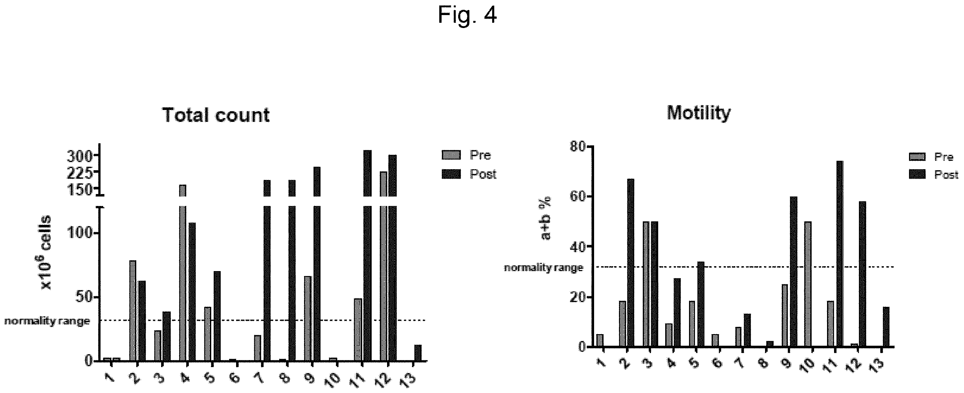

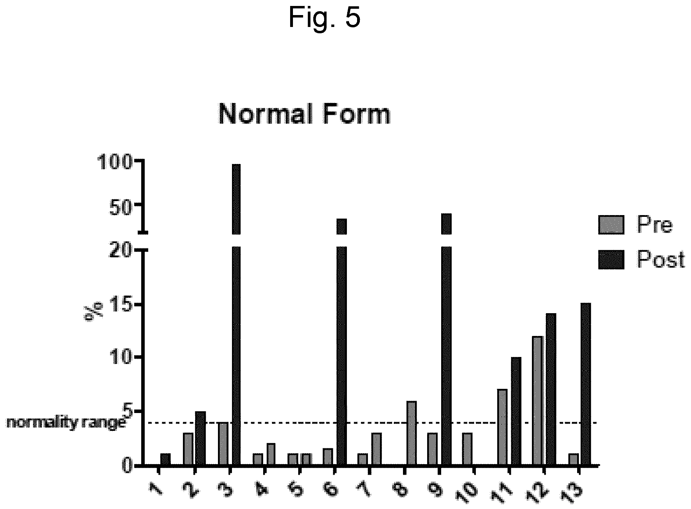

figures 3 to 5 show the graphs of the pre- and post-treatment seminal parameter values for each patient reported in Table 2;

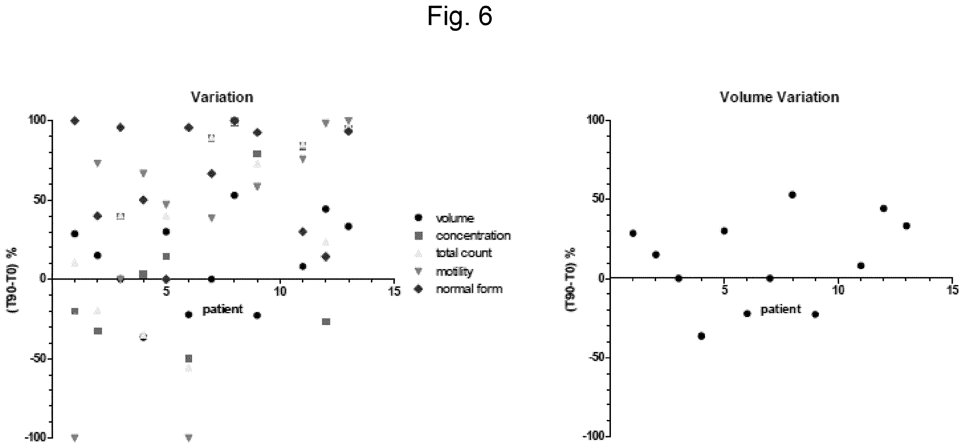

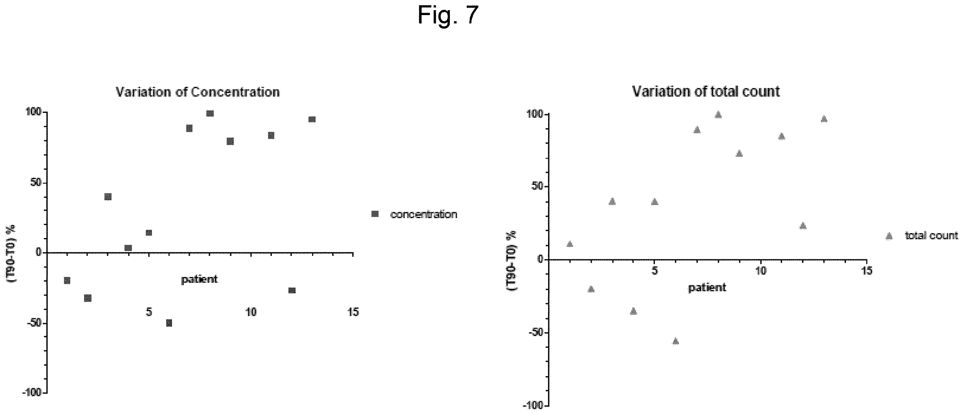

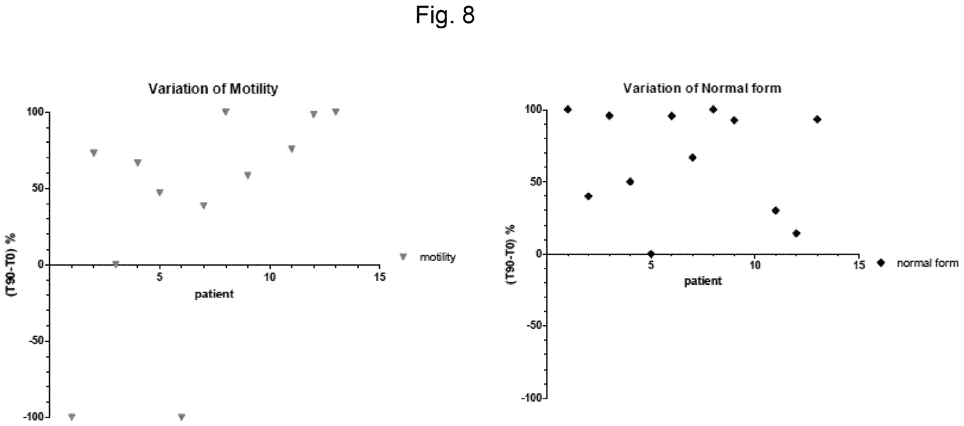

figures 6 to 8 show the graphs depicting the variation of the data between pre- and post-treatment for each patient shown in Table 3.

### Object of the invention

In a first object, the present invention describes a preparation comprising verbascoside, teupolioside and echinacoside.

In a second object of the invention, the medical use of such a preparation is described.

In accordance with a particular aspect, such medical use is for the treatment of male infertility.

In a third object, the use of the preparation of the invention for improving the spontaneous pregnancy rate is described.

### Detailed description of the invention

### Verbascoside

Verbascoside (also known as acteoside) is a secondary metabolite produced by the plant as a defense agent against parasites and insects.

Verbascoside is a phenylpropanoid, characterized by a caffeic acid residue and a hydroxytyrosol residue bound to a glucopyranoside on opposite sides of the sugar chain through an ester and glycosidic bond, respectively:

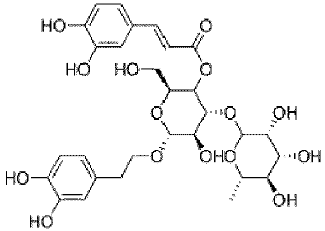

### Teupolioside

Teupolioside (also known as Lamiuside "A") is a secondary metabolite produced by the plant for defensive purposes to protect itself above all from UV radiation. It is a compound characterized by a scarce presence in nature.

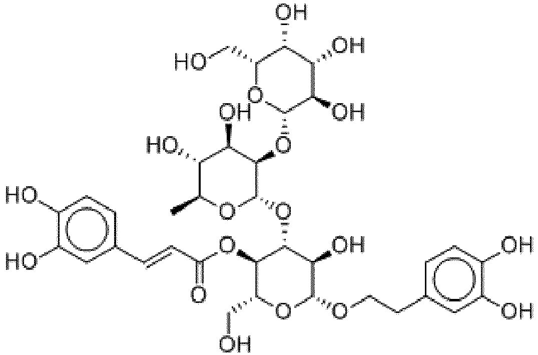

### Echinacoside

Echinacoside is a compound with a phenylpropanoid structure of secondary metabolism produced by the plant for defense against pathogens; it is a natural glycoside of caffeic acid which consists of a trisaccharide formed by two molecules of glucose and one of rhamnose.

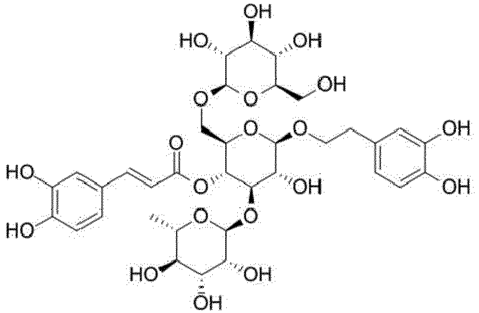

### Seminal fluid evaluation parameters

The following parameters are used in the evaluation of seminal fluid quality (WHO 2010).

Normozoospermia: is the normal condition of seminal parameters.

Oligozoospermia: is the lower sperm concentration condition.

Cryptozoospermia: is the sperm concentration condition <100,000 sperm/ml.

Azoospermia: absence of spermatozoa in the ejaculate.

Necrozoospermia: reduced sperm vitality condition (<58%).

Asthenozoospermia: is the least progressive motility condition (PR) (<32%).

Teratozoospermia: is the lower concentration condition of sperm with normal morphology.

Hypospermia: is the condition of reduced sperm volume in the ejaculate.

In accordance with a first object of the invention, a preparation comprising verbascoside, teupolioside and echinacoside is described.

For the purposes of the present invention, the verbascoside is represented by a verbascoside extract titrated to 10%.

In a preferred aspect, the verbascoside is obtained from plants of the *Verbenaceae* family.

In an even more preferred aspect, the verbascoside is obtained from *Lippia citrodora.*

In particular, *Lippia citrodora* is synonymous with *Aloysia triphylla, Verbena citriodora* and *Verbena triphyilla.*

More in particular, the verbascoside can be obtained from plant cells grown in suspension.

An example of a verbascoside extract titrated to 10% is the product ACTEOS 10P (ABResearch S.r.1.).

For the purposes of the present invention, the teupolioside is represented by a teupolioside extract titrated to 40%.

In a preferred aspect, the teupolioside is obtained from plants of the *Ajugoidae* family.

In an even more preferred aspect, the teupolioside is obtained from *Ajuga reptans.*

More in particular, the teupolioside can be obtained from plant cells grown in suspension.

An example of a teupolioside extract titrated to 40% is the product TEUPOL 40P (ABResearch S.r.1.).

For the purposes of the present invention, the echinacoside is represented by an echinacoside extract titrated to 4%.

In a preferred aspect, the echinacoside is obtained from plants of the *Asteraceae* family.

In an even more preferred aspect, the echinacoside is obtained from *Echinacea angustifolia.*

More in particular, the echinacoside can be obtained from plant cells grown in suspension.

According to an aspect of the present invention, the described preparation can further comprise dicaffeoylquinic acid and/or chlorogenic acid.

In particular, the dicaffeoylquinic acid and chlorogenic acid can be comprised in the echinacoside extract.

An example of an echinacoside extract titrated to 4% is the product EchiPLUS (ABResearch S.r.1.).

Within such an extract, the dicaffeoylquinic acid can be comprised with a titer of about 5%, while the chlorogenic acid with a titer of about 1%.

In accordance with a particular embodiment, the preparation of the invention comprises verbascoside, said teupolioside and said echinacoside in a preferred weight ratio of about 3:1:1.

In accordance with the present patent application, a preparation according to the invention comprises:

| | |
|---|---|
| Verbascoside | 5-100 mg |
| Teupolioside | 2-50 mg |
| Echinacoside | 2-50 mg |

According to a preferred aspect, a preparation of the invention comprises:

| | |
|---|---|
| Verbascoside | 10-50 mg |
| Teupolioside | 2-15 mg |
| Echinacoside | 2-15 mg |

and according to an even more preferred aspect, it comprises:

| | |
|---|---|
| Verbascoside | 10-20 mg |
| Teupolioside | 2-8 mg |
| Echinacoside | 2-8 mg |

For the purposes of the present invention, a particularly preferred preparation has the following composition:

| | |
|---|---|
| Verbascoside extract titrated to 10% | 150 mg |
| Teupolioside extract titrated to 40% | 12.5 mg |
| Echinacoside extract titrated at 4% | 125 mg |

In a second object, the present patent application describes the preparation comprising verbascoside, teupolioside and echinacoside as described above, for medical use.

In a preferred aspect, such a preparation is described for medical use in the treatment of male infertility.

According to a first aspect, in the treatment of male infertility, the preparation of the invention has been shown to increase the volume of the seminal fluid produced.

In a second aspect, in the treatment of male infertility, the preparation of the invention has been shown to increase the concentration of spermatozoa in the seminal fluid.

In a third aspect, in the treatment of male infertility, the preparation of the invention has been shown to increase the total spermatozoa count.

In a fourth aspect, in the treatment of male infertility, the preparation of the invention has been shown to increase spermatozoa motility.

According to a fifth aspect, in the treatment of male infertility, the preparation of the invention has been shown to increase the number of spermatozoa having a normal head form.

For the purposes of the present patent application, the preparation of the invention allows the improvement of one or more of the following properties of seminal fluid: volume, concentration, total count, motility (asthenozoospermia), normal shape (teratozoospermia).

Advantageously, the preparation of the invention has shown that it does not cause changes in the hormonal level of one or more of the following hormones: testosterone, FSH (follicle-stimulating hormone), LH (luteinizing hormone) and PRL (prolactin).

According to a preferred aspect, all the hormonal values of testosterone, FSH (follicle-stimulating hormone), LH (luteinizing hormone) and PRL (prolactin) remain unchanged.

In another aspect, in the treatment of male infertility, the preparation of the invention has been shown to reduce the number of clinical alterations in a patient's seminal fluid.

In the context of the treatment of male infertility, the preparation of the invention is preferably administered twice a day and the treatment period preferably lasts at least 3 months.

In a third object of the present patent application, the use of the preparation of the invention for improving the spontaneous pregnancy rate is described.

The invention will be further described with reference to the results obtained and collected in the following tables.

In particular, a composition according to the invention was administered to enrolled patients twice a day (every 12 hours) for 3 months.

The following Table 1 shows the evaluation of clinical improvement through the measurement of the frequencies of the altered pathophysiological and clinical features of the enrolled patients. The normal level is that defined by (WHO).

| **Clinical diagnosis** | **Pre-treatment no. altered patients/total patients (%)** | **Post-treatment no patients/tot. patients (%)** |
|---|---|---|
| Hypoposia | 2/13 (15.4) | 1/12 (8.3) |
| Oligozoospermia | 8/13 (61.5) | 3/12 (25.0) |
| Asthenospermia | 10/13 (76.9) | 4/12 (33.3) |
| Teratozoospermia | 10/13 (76.9) | 4/12 (33.3) |
| Crypto- or azoospermia | 2/13 (15.4) | 1/12 (8.3) |

The following Table 2 summarizes the mean values of the individual seminal parameters considered.

| | **Pre-treatment median (q1-q3)** | **Post-treatment median (q1-q3)** |
|---|---|---|
| Patients (total no.) | n=13 | n=12 |
| Age (years) | mean ± sd: 37.6 ± 7.4 | |
| Volume (ml) | 2.9 (1.7-5.0) | 3.5 (1.8-6.8) |
| Concentration (x10⁶ cell/ml) | 7.5 (1.0-22.1) | 31.0 (4.7-79.3) |
| Total count (x10⁶ cells) | 23.0 (1.2-72.0) | 89.0 (18.6-229.8) |
| Motility (a+b %) | 18.0 (5.0-25.0) | 34.0 (13.0-60.0) |
| Normal form (%) | 2.3 (1.0-3.8) | 8.0 (2.3-30.0) |

Table 2 shows a median value above the WHO-defined normal level after treatment.

The following Table 3 shows the variation of each seminal parameter between pre- and post-treatment in the enrolled patients.

| **Condition** | **Volume no. patients (%)** | **Concentrati on no. patients (%)** | **Total count no. patients (%)** | **Motility (a+b) no. patients (%)** | **Normal form no. patients (%)** |
|---|---|---|---|---|---|
| **Improvement** | 7 (58.3) | 8 (66.7) | 9 (75.0) | 9 (75.0) | 11 (91.7%) |
| **No improvement/ aggravation** | 5 (41.7) | 4 (33.3) | 3 (25.0) | 3 (25.0) | 1 (8.3) |
| **Tot. patients** | 12 (100.0) | 12 (100.0) | 12 (100.0) | 12 (100.0) | 12 (100.0) |

From the description provided above, the several advantages offered by the present invention will be apparent to those skilled in the art.

The studies carried out have shown:
- an improvement in clinical diagnosis: 3 normospermic patients at the end of treatment and 1 declared pregnancy;
- the reduction in the number of clinical alterations in seminal fluid per patient;
- the improvement of all the seminal parameters considered;
- statistically significant improvement of: total count, rapid motility (%) and normal head form (%) of the spermatozoa:
- the distribution of cases exceeds the normal range for each parameter (concentration, total count, rapid motility and normal head form);
- in at least 58% of patients a seminal parameter improved;
- in at least 75% of patients the number of spermatozoa increased (with a consequent increase in the probability of fertilization);
- in at least 75% of patients the % of spermatozoa with rapid motility (important for fertilization) increased;
- in at least 92% of patients, the % of spermatozoa with normal head form increased (with consequent increase in the probability of fertilization).

Furthermore, the composition described was shown to be well tolerated by all study participants, especially in the intestine, and no adverse reactions, side effects or poor tolerance of the product were reported.

Finally, benefits far beyond expectations were demonstrated, especially in patients with astheno- or oligo-zoospermia, especially severe ones, and in patients with a probable cause of obstruction at the level of the seminiferous tubules.

## Claims

1. A preparation comprising verbascoside, teupolioside and echinacoside.

2. A pharmaceutical preparation according to the preceding claim, wherein verbascoside is a preparation titrated to 10% in verbascoside.

3. A pharmaceutical preparation according to the preceding claim, wherein teupolioside is a preparation titrated to 40% in teupolioside.

4. A pharmaceutical preparation according to the preceding claim, wherein echinacoside is a preparation titrated to 4% in echinacoside.

5. A preparation according to any one of the preceding claims, wherein said verbascoside, said teupolioside and said echinacoside are preferably present in a weight ratio of about 3:1:1.

6. A preparation according to any one of the preceding claims, further comprising dicaffeoylquinic acid and/or chlorogenic acid.

7. A preparation according to any one of the preceding claims, wherein verbascoside is obtained from plants of the *Verbenaceae* family.

8. A preparation according to any one of the preceding claims, wherein teupolioside is obtained from plants of the *Ajugoidae* family.

9. A preparation according to any one of the preceding claims, wherein echinacoside is obtained from plants of the *Asteraceae* family.

10. A preparation according to any one of the preceding claims, comprising 5-100 mg, preferably 10-50 mg verbascoside, 2-50 mg and preferably 2-15 mg teupolioside, and 2-50 mg and preferably 2-15 mg echinacoside.

11. A preparation according to the preceding claim, comprising 10-20 mg verbascoside, 2-8 mg teupolioside, and 2-8 mg echinacoside.

12. A preparation according to any one of the preceding claims, for medical use.

13. A preparation according to any one of the preceding claims for medical use in the treatment of male infertility.

14. A preparation according to the preceding claim 12 or 13, for medical use in the treatment of male infertility, wherein one or more of the following properties of the seminal fluid is improved: volume, concentration, total count, motility, normal form.

15. A preparation according to any one of the preceding claims 12 to 14, which is administered twice a day for a period of 3 months.

16. Use of a preparation according to any one of the preceding claims 1 to 11, for the improvement of the spontaneous pregnancy rate.
